**Europäisches Patentamt**

(19) **European Patent Office**

**Office européen des brevets**

(11) Veröffentlichungsnummer: **0 088 420**

**B1**

(12) # EUROPÄISCHE PATENTSCHRIFT

(45) Veröffentlichungstag der Patentschrift: **24.09.86**

(51) Int. Cl.⁴: **C 12 Q 1/60**

(21) Anmeldenummer: **83102231.4**

(22) Anmeldetag: **07.03.83**

(54) Verfahren zur spezifischen Bestimmung des Cholesterins der LDL-Fraktion im Serum.

(30) Priorität: **08.03.82 DE 3208253**

(43) Veröffentlichungstag der Anmeldung:
**14.09.83 Patentblatt 83/37**

(45) Bekanntmachung des Hinweises auf die Patenterteilung:
**24.09.86 Patentblatt 86/39**

(84) Benannte Vertragsstaaten:
**AT BE CH DE FR GB IT LI LU NL SE**

(56) Entgegenhaltungen:
**EP-A-0 035 211
DE-B-2 558 536
US-A-4 186 251
US-A-4 226 713**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Ziegenhorn, Joachim, Dr. rer. nat., Ina-Seidel- Weg 1, D-8130 Starnberg (DE)**
Erfinder: **Röder, Albert, Dr. rer. nat., Bahnhofstrasse 41, D-8124 Seeshaupt (DE)**
Erfinder: **Bartl, Knut, Dr. rer. nat., Am Westend 6, D-8121 Wilzhofen (DE)**
Erfinder: **Wehmeyer, Gunter, Dr. rer. nat., Oberer Seeweg 27, D-8130 Starnberg (DE)**

(74) Vertreter: **Weickmann, Heinrich, Dipl.- Ing., Patentanwälte Dipl.- Ing. H.Weickmann Dipl.-Phys.Dr. K.Fincke Dipl.- Ing. F.A.Weickmann Dipl.- Chem. B. Huber Dr.- Ing. H. Liska Dipl.-Phys.Dr. J. Prechtel Postfach 860820, D-8000 München 86 (DE)**

## Beschreibung

Die Erfindung betrifft ein Verfahren zur spezifischen Bestimmung des Cholesterins der LDL-Fraktion in Gegenwart der HDL-Fraktion der Lipoproteine des Serums.

Die Bestimmung der LDL-Fraktion (Low Density Lipoprotein), auch β-Lipoproteinfraktion genannt, hat für die differenzierte Diagnose einer Lipidstoffwechselstörung erhebliche Bedeutung erlangt.

Hypercholesterinämie und Hypertriglyceridämie begünstigen die Entstehung der Atherosklerose und des Herzinfarkts. Die Bestimmung von Cholesterin und Triglyceriden im Serum gehören daher zu den am häufigsten durchgeführten Tests im klinisch-chemischen Routinelabor.

Zahlreiche Untersuchungen zum Fettstoffwechsel kommen zu dem Schluß, daß das individuelle Coronarrisiko besser erfaßt werden kann, wenn nicht nur die Veränderung im Triglycerid- und Cholesterinspiegel bestimmt, sondern die zugrundeliegenden pathologischen Verschiebungen im Lipoproteinmuster ermittelt werden (Münch. med. Woschr. 121 (1979) 1639).

- Die bekannten Plasmalipoproteine enthalten einen unterschiedlich hohen Anteil an Protein, Phospholipiden, Cholesterin und Triglyceriden. Sie lassen sich aufgrund ihres Verhaltens (unterschiedliche Dichte) in der analytischen Ultrazentrifuge in drei verschiedene Klassen unterteilen:

Prä-β-Lipoprotein = VLDL (Very Low Density Lipoprotein)

β-Lipoprotein = LDL (Low Density Lipoprotein)

α-Lipoprotein = HDL (High Density Lipoprotein).

Die Untersuchung der Funktion der Lipoproteine ergab, daß LDL innerhalb der Lipoproteine die entscheidende atherogene Komponente darstellt, bei deren Erhöhung im Blut ein gesteigertes Risiko für eine coronare Herzkrankheit vorliegt. Die frühzeitige Erkennung und Bekämpfung dieses Zustands ist von großer Bedeutung. Es besteht daher ein Bedarf nach einem praktikablen Verfahren zur quantitativen Bestimmung der LDL-Konzentration im Serum und Plasma.

Bisher wurden für die Bestimmung der LDL-Lipoproteinfraktion im wesentlichen drei Methoden eingesetzt, die jedoch Nachteile besitzen:

## 1. Ultrazentrifugation

Dieses Verfahren ist für ein Routinelabor nicht geeignet, da man hierzu eine spezielle Geräteausrüstung benötigt und die Durchführung eine äußerst sorgfältige Arbeitstechnik und einen sehr hohen Zeitaufwand (mehrtägiges Zentrifugieren in einer Ultrazentrifuge) erfordert. Somit blieb dieses Analysenverfahren bisher nur dem Labor der forschenden Medizin vorbehalten.

## 2. Fällungsreaktion

Der LDL-Gehalt kann durch fraktionierte Fällung mit einem Polyanion, wie Heparin-Na oder Dextransulfat, und einem zweiwertigen Kation, wie $Ca^{++r}$, $Mn^{++}$, $Mg^{++}$, ebenfalls bestimmt werden. Die Lipoproteine lassen sich nämlich mit steigender Konzentration des Polyanions in der folgenden Reihenfolge ausfällen: VLDL, LDL und HDL. Dieses Verfahren erfordert jedoch zwei Arbeitsschritte und ist somit unpraktikabel und nicht automatisierbar: VLDL wird in einem ersten Fällungsschritt abgetrennt, anschließend wird durch Erhöhung der Konzentration des Fällungsmittels die LDL-Lipoproteinfraktion gefällt und turbidimetrisch bestimmt (H. Okabe, X. Int. Congr. of Clin. Chem., Mexico (1978)).

## 3. Bestimmung der LDL-Konzentration über die FriedewaldFormel

Bei diesem Verfahren bestimmt man den Triglycerid- und den Gesamtcholesteringehalt sowie durch Ausfällen von VLDL und LDL den HDL-Cholesteringehalt der Probe und berechnet daraus den Gehalt an LDL-Cholesterin nach dem Verfahren von Friedewald, Clin. Chem. 18 (1972) 499. Dieses umständliche Verfahren ist ebenfalls unpraktikabel.

## 4. Elektrophoretische Trennung und Polyanionenfällung

Dieses Verfahren jedoch ist zeitaufwendig und erfordert eine eigene Elektrophorese-Apparatur sowie ein Densitometer zur Auswertung (Lab. Med. 1 (1977) 145).

Ein Verfahren, welches die oben geschilderten Nachteile vermeidet, ist in der DE-OS 30 07 764.6 beschrieben. Dieses Verfahren beruht auf der direkten turbidimetrischen Bestimmung der LDL-Fraktion in Körperflüssigkeiten durch Fällung mit einem Polyanion und einem zweiwertigen Kation aus wässriger Lösung, bei dem man die Fällung in Gegenwart eines Komplexbildners bei einem pH-Wert zwischen 7 und 9 durchführt. Dieses Verfahren bringt zwar einen erheblichen technischen Fortschritt gegenüber den oben erwähnten älteren Verfahren und ist insbesondere automatisierbar und daher für das Routinelabor geeignet. Da es jedoch zwingend eine turbidimetrische Bestimmung erfordert, für die nicht alle Labors eingerichtet sind, bestand daneben noch Bedarf nach einem vereinfachten Verfahren, welches als Farbtest sowohl mit üblichen Photometern als auch mit Analysenautomaten durchführbar ist.

Ein Vorteil des erfindungsgemäßen Verfahrens liegt darin, daß im Unterschied zum LDL-Gesamtpartikel mit dem LDL-Cholesterin ein Parameter bestimmt wird, auf dessen Quantifizierung die bisherigen epidemiolog. üntersuchungen überwiegend basieren.

Daher liegen für diesen Parameter zuverlässigere "Normalwerte" vor als für den LDL-Gehalt bzw. HDL-Gehalt insgesamt. Bisher mußte zur Bestimmung des Choleteringehalts in der LDL-Fraktion daher letztere erst isoliert und dann in einem gesonderten Schritt das Cholesterin bestimmt werden. Die direkte Bestimmung des Cholesterins in der LDL-Fraktion ohne vorherige Fraktionstrennung ist daher nicht nur aus Praktikabilitätsgründen erwünscht, sondern liefert direkt den aussagekräftigsten Parameter.

Der Erfindung liegt daher die Aufgabe zugrunde, ein Verfahren zu schaffen, welches ohne Fällungsreaktion und ohne Fraktionstrennung eine direkte enzymatische Bestimmung des in der LDL-Fraktion enthaltenen Cholesterins gestattet und welches auch als Farbtest durchgeführt werden kann.

Gelöst wird diese Aufgabe erfindungsgemäß durch ein Verfahren, welches den überraschenden Effekt ausnützt, daß unter bestimmten Bedingungen die enzymatische Erfassung des in den Lipoproteinen vorliegenden Cholesterinanteils in der LDL-Fraktion wesentlich rascher verläuft als in der HDL-Fraktion.

Das erfindungsgemäße Verfahren zur spezifischen Bestimmung des Cholesterins der LDL-Fraktion in Gegenwart der HDL-Fraktion der Lipoproteine des Serums durch Einwirkung von Cholesterinesterase zur Freisetzung des Cholesterins und Oxidation des freigesetzten Cholesterins mit Cholesterinoxidase und Sauerstoff unter Bildung von $H_2O_2$ und Cholestenon und kinetische Messung der Veränderung eines Reaktionspartners der Oxidasereaktion, insbesondere der $H_2O_2$-Bildung, ist daher dadurch gekennzeichnet, daß die Messung in einem vorbestimmten Zeitintervall durchgeführt wird und in der Reaktionslösung eine Tensidkonzentration von 0,01 bis 1,5 mmol/l, eine Cholesterinesterase-Konzentration von 0,1 bis 30 U/ml und ein pH-Wert von 6,5 bis 8,0 eingestellt werden.

Die Erfindung beruht also darauf, daß genau aufeinander abgestimmte Konzentrationsverhältnisse von Tensiden und Cholesterinesterasen und ein bestimmter pH-Wert eingestellt werden.

Unter den vorstehend angegebenen Bedingungen verläuft überraschenderweise die enzymatische Erfassung des Cholesterinanteils in der HDL-Fraktion mit konstanter Geschwindigkeit, bis zu etwa 150 mg/100 ml unabhängig von der HDL-Konzentration (Normalbereich 35 bis 55 mg/100 ml), während die Reaktionsgeschwindigkeit des Cholesterinanteils in der LDL-Fraktion linear von deren Konzentration abhängt. Bei einer kinetischen Messung ist der dabei erhaltene Meßwert direkt proportional zur LDL-Cholesterin-Konzentration im Reaktionsansatz.

Die Bestimmung des freien Cholesterins mittels Cholesterinoxidase unter Bildung von $H_2O_2$ und Cholestenon und die kinetische Bestimmung der Veränderung eines Reaktionspartners dieser Oxidationsreaktion ist bekannt, beispielsweise aus der deutschen Auslegeschrift 25 58 536. Als Meßparameter kann dabei sowohl die Abnahme der Sauerstoffkonzentration als auch die Bildung von Cholestenon bzw. $H_2O_2$ verwendet werden. Bevorzugt wird gebildetes $H_2O_2$ bestimmt unter Anwendung der dem Fachmann geläufigen colorimetrischen Methoden der $H_2O_2$-Bestimmung. Eine typische $H_2O_2$-Bestimmungsmethode, die fur die vorliegende Erfindung besonders geeignet ist, beruht auf der Farbreaktion von Phenol oder einem Phenolderivat mit 4 Aminoantipyrin oder einem Derivat davon in Anwesenheit von Peroxidase. Diese Bestimmungen sind dem Fachmann geläufig und brauchen hier nicht näher beschrieben zu werden.

Als Cholesterinoxidase eignen sich die handelsüblichen Präparate. Gleiches gilt für die Cholesterinesterase. Bevorzugt werden Cholesterinesterasepräparate aus Pseudomonaden oder Candidastämmen. Derartige Enzympräparate sind bekannt und handelsüblich.

Erfindungswesentlich ist die kinetische Durchführung der Messung in einem vorbestimmten Zeitintervall sowie die Einhaltung einer bestimmten Tensidkonzentration, einer bestimmten Cholesterinesterase-Aktivität und eines bestimmten pH-Wert-Bereiches.

Grundsätzlich lassen sich im Rahmen der Erfindung Tenside aus den bekannten Gruppen, nämlich der nichtionischen, der kationischen, der anionischen und der Gallensäurederivate verwenden. Die jeweils optimalen Konzentrationen sind jedoch bei diesen Gruppen etwas verschieden und im Falle der Gallensäurederivate und der kationischen Tenside wird vorzugsweise auch innerhalb eines speziellen und engeren pH-Wert-Bereiches gearbeitet.

Bei den nichtionischen Tensiden, bei denen es sich im wesentlichen um die Alkyl- und Alkyl-Aryl-Ester- und -Äther von Polyäthylenoxiden handelt, liegt der bevorzugte Konzentrationsbereich zwischen 0,01 und 0,3 mmol/l. Besonders bevorzugt wird der Bereich von 0,06 bis 0,18 mmol/l.

Bei den anionischen Tensiden, wie beispielsweise Natriumdioctylsulfosuccinat liegt die bevorzugte Konzentration zwischen 0,01 und 0,6 mmol/l; besonders bevorzugt wird der Bereich von 0,08 bis 0,3 mmol/l.

Bei den Gallensäurederivaten, die an sich zu den anionischen Tensiden gezählt werden können, ist der bevorzugte Bereich besonders breit und liegt zwischen 0,05 und 1,0 mmol/l. In

diesem Fall wird jedoch vorzugsweise ein pH-Bereich zwischen 7,6 und 8,0 eingehalten. Bevorzugte Substanz ist Desoxicholat, z. B. als Na-Salz.

Bei kationischen Tensiden, wie CTAB (Cetyltrimethylammoniumbromid), liegt der bevorzugte Bereich zwischen 0,02 und 0,8 mmol/l. Besonders bevorzugt ist der Bereich von 0,08 bis 0,35 mmol/l. Hier wird vorzugsweise ein pH-Wert zwischen 6,8 und 7,2 eingehalten.

Im Rahmen der Erfindung können auch Mischungen von Tensiden verwendet werden, beispielsweise Mischungen von nichtionischen Tensiden und Tensiden aus der Gallensäuregruppe.

Zur Einstellung des erforderlichen pH-Wert-Bereiches werden jeweils die in den angegebenen Bereichen wirksamen Puffersubstanzen zugesetzt. An sich bestehen hinsichtlich der verwendbaren Puffersubstanzen keine Einschränkungen, bevorzugt wird jedoch Phosphatpuffer und insbesondere Tris/HCl-Puffer. Die Pufferkonzentration kann im allgemeinen zwischen etwa 20 und etwa 500 mmol/l liegen, bevorzugt wird der Bereich von 50 bis 200 mmol/l.

Der Meßzeitraum kann relativ breit gefaßt werden. Bevorzugt wird jedoch die Messung im Bereich zwischen 0,5 und 10 Minuten nach dem Start der Reaktion durchgeführt, wobei z. B. im Falle eines Farbtests die Extinktionsdifferenz im Meßintervall bestimmt wird, also beispielsweise die Differenz zwischen der Extinktion 0,5 und 10 Minuten nach Reaktionsstart. Der Meßzeitraum läßt sich jedoch auch wesentlich verkürzen.

Für die Durchführung der Erfindung können handelsübliche Reagenzien auf Basis eines Gehaltes von Cholesterinoxidase und Cholesterinesterase verwendet werden, soweit sie nicht einen pH-Wert außerhalb des für die Erfindung zulässigen Bereichs ergeben. Diesen Reagenzien wird dann das gewählte Tensid in der durch die Erfindung gelehrten Konzentration zugesetzt. Die bevorzugten Bereiche für die einzelnen Reagenzbestandteile liegen im Falle des oben schon erwähnten Farbreagenz bei

100 bis 5.000 U/l Cholesterinoxidase,
100 bis 30.000 U/l Cholesterinesterase,
100 bis 5.000 U/l Peroxidase,
0,5 bis 10 mmol/l 4-Aminoantipyrin,
5 bis 20 mmol/l Phenol bzw. Phenolderivat
sowie Tensid und Puffer wie oben schon näher erläutert.

Die Erfindung ermöglicht es, direkt den am meisten interessierenden Parameter, nämlich den Cholesteringehalt in der LDL-Fraktion, zu bestimmen ohne vorherige Trennung der Lipoproteidfraktionen, ohne Fällungsreaktion und ohne Zentrifugieren. Das Verfahren läßt sich einfach und schnell durchführen und erfaßt spezifisch nur das Cholesterin in der LDL-Fraktion in in Gegenwart der HDL-Fraktion.

Die folgenden Beispiele erläutern dies weiter.

Die Beispiele wurden alle unter Verwendung eines GEMSAEC-Analysenautomaten durchgeführt. Um die Vergleichbarkeit zu erzielen, wurde stets in 100 mmol/l Tris/HCl-Puffer pH 7,5 gearbeitet mit einer Konzentration von 600 U/l Cholesterinoxidase, 2.000 U/l Peroxidase, 10 mmol/l Phenol und 1 mmol/l 4-Aminoantipyrin.

**Beispie 1**

Frisches Humanserum wurde sowohl mit physiologischer Kochsalzlösung allein als auch zusammen mit verschiedenen Mengen LDL, die zueinander in einem linearen Mengenverhältnis standen, so gemischt, daß jeweils eine 1:1 Verdünnung des Serums resultierte. Die zugesetzte LDL- Fraktion war durch Ultrazentrifugation von Serum und Isolierung der entsprechenden Bande gewonnen worden.

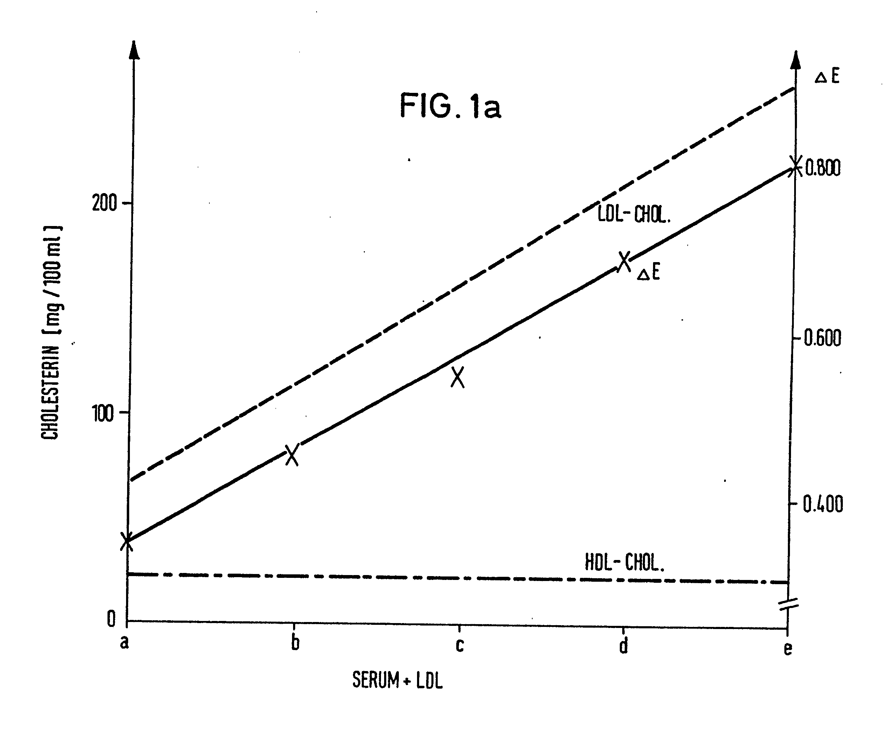

Dem so erhaltenen, mit LDL aufgestockten Serum wurden die oben erwähnten Mengen an Cholesterinoxidase, Peroxidase, Phenol und 4-Aminoantipyrin sowie Tris/HCl zugesetzt. Außerdem wurde Cholesterinesterase aus Pseudomonas auf 10 U/ml und das anionische Tensid Aerosol OT der Firma American Cyanamid bis 0,11 mmol/l zugegeben. Dann wurde die Extinktion nach 0,5 und nach 10 Minuten gemessen. Die Ergebnisse zeigt Fig. 1a der Zeichnung in graphischer Darstellung. Man erkennt daraus, daß zwischen Meßwert und Konzentration an LDL-Cholesterin eine lineare Korrelation besteht. In der Figur ist links die Cholesterinkonzentration in mg pro 100 ml, rechts die Extinktionsdifferenz angegeben.

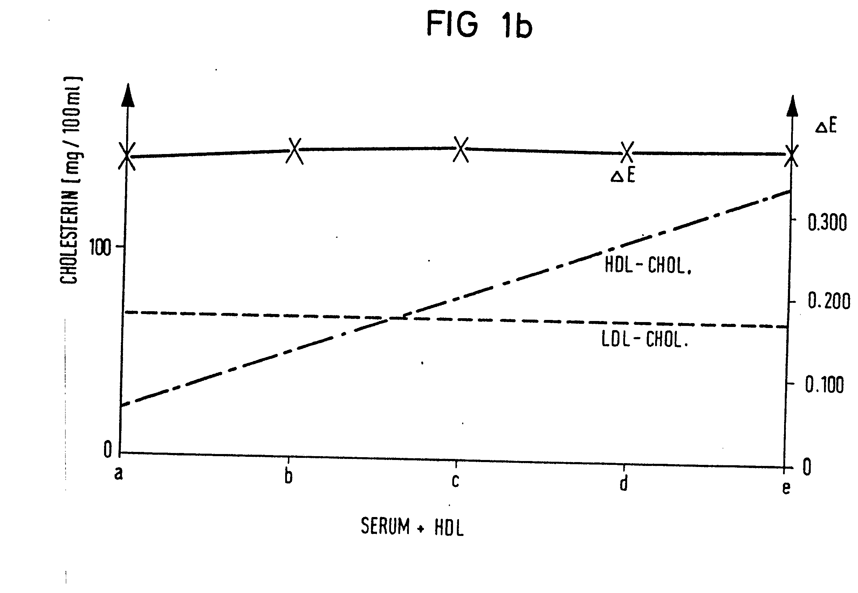

Der oben beschriebene Versuch wurde wiederholt, jedoch wurde das Humanserum nicht mit einer LDL-Fraktion sondern mit einer HDL-Fraktion aufgestockt, wobei wegen der erheblich niedrigeren HDL-Konzentration im Serum andere Konzentrationsverhältnisse gewählt werden. Die Ergebnisse sind in Fig. 1b der Zeichnung graphisch dargestellt. Man erkennt, daß keine lineare Korrelation zwischen Meßwert und der Konzentration an HDL-Cholesterin besteht sondern die Extinktionsdifferenz dem LDL-Gehalt des Ausgangsserums entspricht.

**Beispiel 2**

Es wurde, wie in Beispiel 1 beschrieben, vorgegangen. Der Gehalt an Cholesterinesterase betrug 10 U/ml. Als Tensid wurde Natriumdesoxycholat auf eine Konzentration von 0,10 mmol/l zugesetzt.

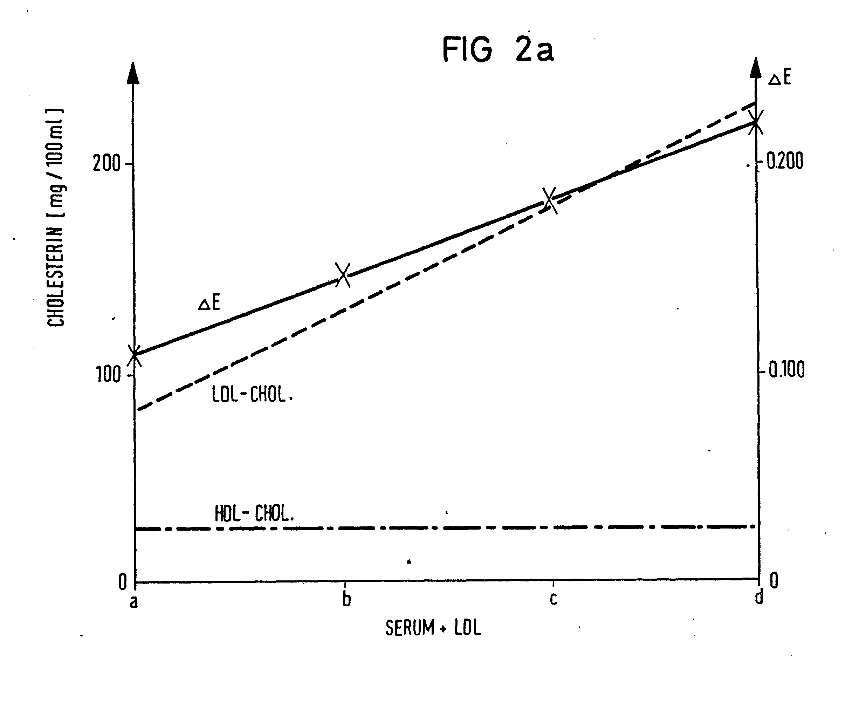

Die Ergebnisse bei dem mit LDL-Fraktion aufgestockten Gemisch sind in Fig. 2a gezeigt. Man erkennt wieder eine lineare Korrelation zwischen Meßwert und LDL-Cholesterin-Konzentration.

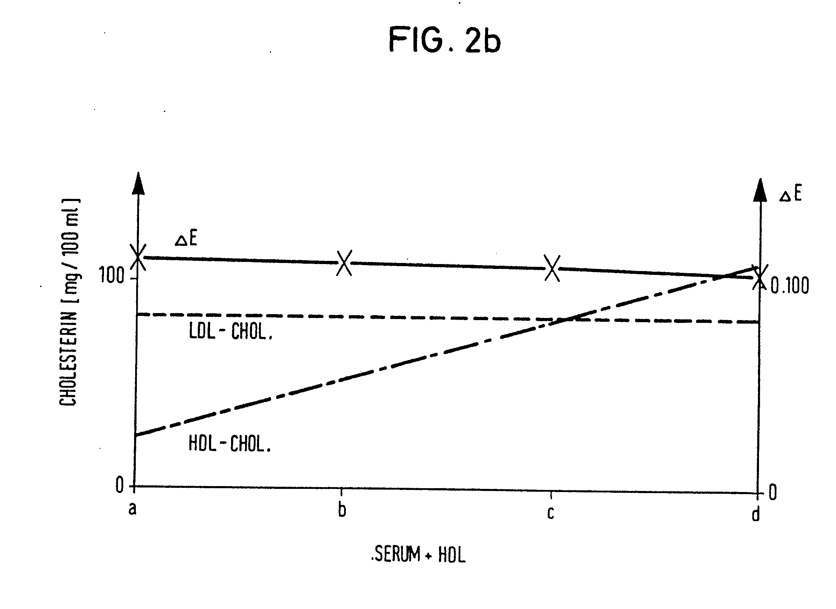

Die bei der Wiederholung des Versuches mit einem mit HDL aufgestockten Humanserum erhaltenen Resultate sind in Fig. 2b dargestellt. Man erkennt, daß hier der Meßwert wiederum der LDL-Konzentration linear korreliert (diese Konzentration war in sämtlichen Proben gleich und entsprach dem Gehalt des 1:1 verdünnten Serums an ursprünglich vorhandenem LDL), während keine Korrelation zur HDL-Konzentration besteht.

## Beispiel 3

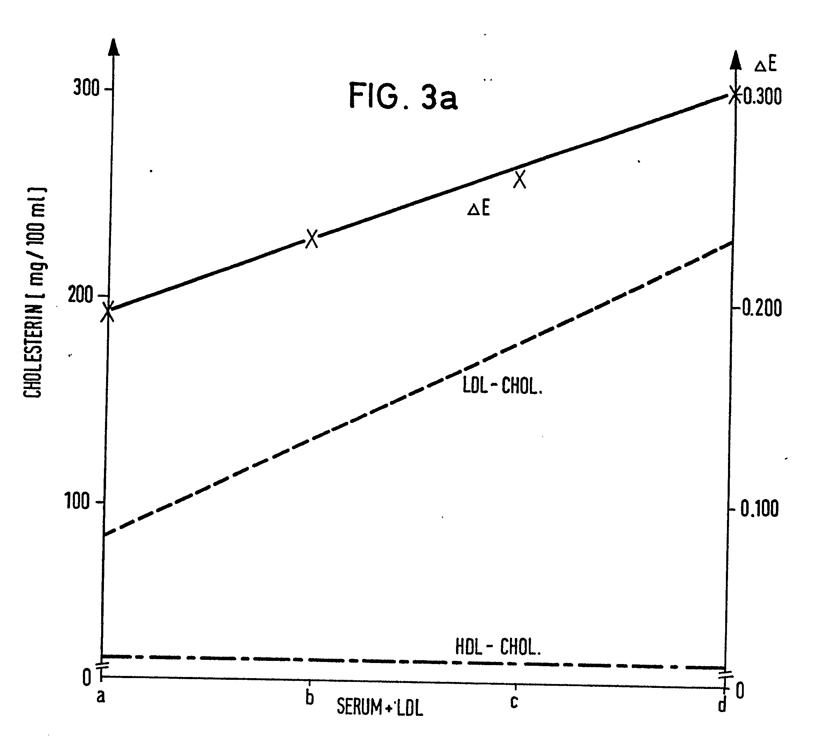

Es wurde wie in Beispiel 1 beschrieben vorgegangen. Der Cholesterinesterasegehalt in der Meßlösung war 2 U/ml. Als Tensid wurde das kationische Cetyltrimethylammoniumbromid auf eine Konzentration von 0,166 mmol/l zugegeben. Die Ergebnisse bei dem mit LDL aufgestockten Humanserum sind in Figur 3a der Zeichnung graphisch dargestellt. Auch hier ist eine lineare Korrelation zwischen Meßwert und LDL-Cholesterin-Konzentration erkennbar.

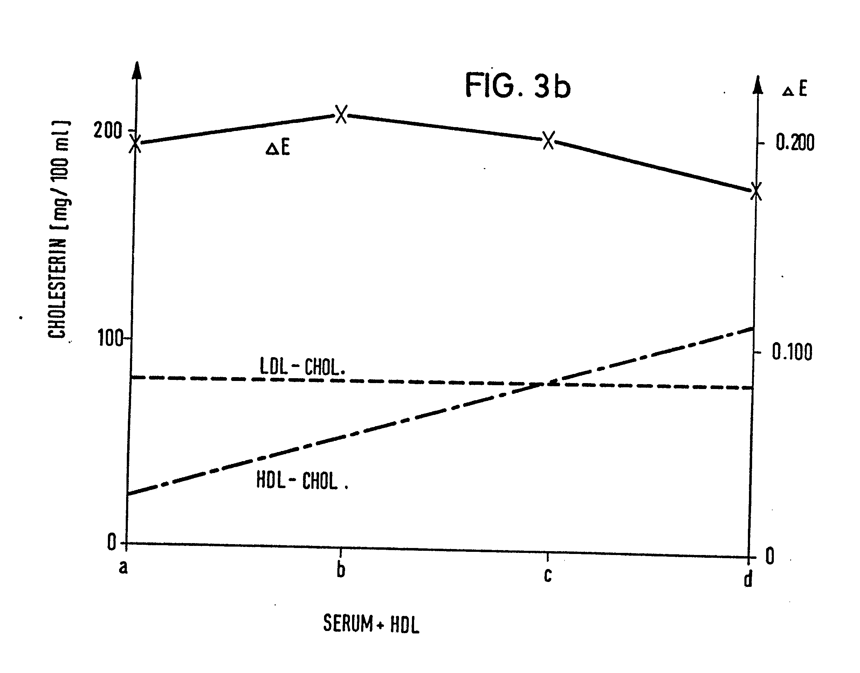

Die Ergebnisse mit dem mit HDL aufgestockten Humanserum sind in Fig. 3b der Zeichnung graphisch dargestellt. Man erkennt wiederum, daß keine Korrelation zwischen dem HDL-Gehalt und dem Meßwert besteht, der mit einer Schwankung von etwa 8 % konstant ist und der ursprünglich im Serum vorhandenen LDL-Menge entspricht.

## Beispiel 4

Die unterschiedliche Reaktion der LDL- und HDL-Fraktion wird an folgendem Beispiel deutlich. Es wurde das in Beispiel 3 beschriebene Reagenzgemisch mit gleichem Tensid und gleicher Tensidkonzentration und mit einer Cholesterinesterasekonzentration von 10 U/ml verwendet. Zu diesem Reagenzgemisch wurden in zwei verschiedenen Ansätzen gleiche Mengen einer isolierten LDL-Fraktion der Konzentration 385 mg Cholesterin pro Deziliter bzw. einer HDL-Fraktion der Konzentration 226 mg Cholesterin pro Deziliter zugesetzt.

Die mit der LDL-Fraktion erhaltene Extinktionsdifferenz betrug 0,3259, umgerechnet auf 226 mg/Deziliter = 0,1934.

Bei dem mit HDL aufgestockten Serum betrug die Extinktionsdifferenz 0,0176.

Hieraus errechnet sich ein Verhältnis $LDL_{226}/HDL_{226}$ = ca 10:1

## Patentansprüche

1. Verfahren zur spezifischen Bestimmung des Cholesterins der LDL-Fraktion in Gegenwart der HDL-Fraktion der Lipoproteine des Serums durch Einwirkung von Cholesterinesterase zur Freisetzung des Cholesterins und Oxidation des freigesetzten Cholesterins mit Cholesterinoxidase und Sauerstoff unter Bildung von $H_2O_2$ und Cholestenon und kinetische Messung der Veränderung eines Reaktionspartners der Oxidasereaktion, insbesondere der $H_2O_2$-Bildung, dadurchgekennzeichnet, daß die Messung in einem vorbestimmten Zeitintervall durchgeführt wird und in der Reaktionslösung eine Tensidkonzentration von 0,01 bis 1,5 mmol/l, eine Cholesterinesterase-Konzentration von 0,1 bis 30 U/ml und ein pH-Wert von 6,5 bis 8,0 eingestellt werden.

2. Verfahren nach Anspruch 1, dadurch gekennzeichnet, daß ein nichtionisches Tensid in einer Konzentration von 0,01 bis 0,30 mmol/l eingesetzt wird.

3. Verfahren nach Anspruch 2, dadurch gekennzeichnet, daß die Tensidkonzentration 0,06 bis 0,18 mmol/l beträgt.

4. Verfahren nach Anspruch 1, dadurch gekennzeichnet, daß ein Tensid der Gallensäuregruppe in einer Konzentration von 0,01 bis 1,5 mmol/l eingesetzt und der pH-Wert bei 7,2 bis 8,0 gehalten wird.

5. Verfahren nach Anspruch 4, dadurch gekennzeichnet, daß die Tensidkonzentration 0,05 bis 1,10 mmol/l beträgt und der pH-Wert bei 7,6 bis 8,0 gehalten wird.

6. Verfahren nach Anspruch 1, dadurch gekennzeichnet, daß ein kationisches Tensid in einer Konzentration von 0,02 bis 0,80 mmol/l eingesetzt und der pH-Wert auf 6,5 bis 7,6 eingestellt wird.

7. Verfahren nach Anspruch 6, dadurch gekennzeichnet, daß die Tensidkonzentration 0,08 bis 0,35 mmol/l beträgt und der pH-Wert auf 6,8 bis 7,2 eingestellt wird.

8. Verfahren nach Anspruch 1, dadurch gekennzeichnet, daß ein anionisches Tensid in einer Konzentration von 0,01 bis 0,70 mmol/l eingesetzt wird.

9. Verfahren nach Anspruch 8, dadurch gekennzeichnet, daß die Tensidkonzentration 0,05 bis 0,35 mmol/l beträgt.

10. Verfahren nach einem der Ansprüche 1 bis 8, dadurch gekennzeichnet, daß die Messung innerhalb von 0,5 bis 15 Minuten nach Start der Reaktion durchgeführt wird.

11. Verfahren nach einem der vorhergehenden Ansprüche, dadurch gekennzeichnet, daß man Cholesterinoxidase aus Nocardia erythropolis verwendet.

12. Verfahren nach einem der vorhergehenden Ansprüche, dadurch gekennzeichnet, daß man eine Cholesterinesterase aus Candida oder Pseudomonas verwendet.

13. Reagenz zur Durchführung des Verfahrens nach Anspruch 1, dadurch gekennzeichnet, daß es

200 bis 1000 U/l Cholesterinoxidase,
1000 bis 3000 U/l Peroxidase,
2000 bis 10000 U/l Cholesterinesterase,
0,10 bis 0,16 mmol/l Tensid,
2 bis 20 mmol/l Phenol,
0,5 bis 3 mmol/l 4-Aminoantipyrin ml

70 bis 130 mmol/l Tris/HCl pH 7,3 bis 7,7 enthält.

## Claims

1. Process for the specific determination of the cholesterol of the LDL fraction in the presence of the HDL fraction of the lipoproteins of serum by the action of cholesterol esterase for the liberation of the cholesterol and oxidation of the liberated cholesterol with cholesterol oxidase and oxygen with the formation of $H_2O_2$ and cholestenone and kinetic measurement of the change of one of the reaction components of the oxidase reaction, especially of the $H_2O_2$ formation, characterised in that the measurement is carried out in a predetermined time interval and in the reaction solution there are adjusted a tenside concentration of 0.01 to 1.5 mmol/l., a cholesterol esterase concentration of 0.1 to 30 U/ml. and a pH value of 6.5 to 8.0.

2. Process according to claim 1, characterised in that a non-ionic tenside is used in a concentration of 0.01 to 0.30 mmol/l.

3. Process according to claim 2, characterised in that the tenside concentration amounts to 0.06 to 0.18 mmol/l.

4. Process according to claim 1, characterised in that a tenside of the bile acid group is used in a concentration of 0.01 to 1.5 mmol/l. and the pH value is kept at 7.2 to 8.0.

5. Process according to claim 4, characterised in that the tenside concentration amounts to 0.05 to 1.10 mmol/l. and the pH value is kept at 7.6 to 8.0.

6. Process according to claim 1, characterised in that a cationic tenside is used in a concentration of 0.02 to 0.80 mmol/l. and the pH value is adjusted to 6.5 to 7.6.

7. Process according to claim 6, characterised in that the tenside concentration amounts to 0.08 to 0.35 mmol/l. and the pH value is adjusted to 6.8 to 7.2.

8. Process according to claim 1, characterised in that an anionic tenside is used in a concentration of 0.01 to 0.70 mmol/l.

9. Process according to claim 8, characterised in that the anionic tenside concentration amounts to 0.05 to 0.35 mmol/l.

10. Process according to one of claims 1 to 8, characterised in that the measurement is carried out within 0.5 to 15 minutes after the start of the reaction.

11. Process according to one of the preceding claims, characterised in that one uses cholesterol oxidase from Nocardia erythropolis.

12. Process according to one of the preceding claims, characterised in that one uses a cholesterol esterase from Candida or Pseudomonas.

13. Reagent for the carrying out of the process according to claim 1, characterised in that it contains:

200 to 1000 U/l. cholesterol oxidase,
1000 to 3000 U/l. peroxidase,
2000 to 10,000 U/l. cholesterol esterase,
0.10 to 0.16 mmol/l. of tenside,
2 to 20 mmol/l. phenol,
0.5 to 3 mmol/l. 4-aminoantipyrine ml.,
70 to 130 mmol/l. tris/HCl pH 7.3 to 7.7.

## Revendications

Procédé de dosage spécifique du cholestérol de la fraction LDL en présence de la fraction HDL des lipoprotéines du sérum par action de la cholystérolestérase pour la libération du cholestérol et oxydation du cholestérol libéré avec de la cholestéroloxydase et de l'oxygène avec formation de $H_2O_2$ et de cholesténone et mesure cinétique de la modification d'un partenaire réactionnel de la réaction de l'oxydase, en particulier de la formation de $H_2O_2$, caractérisé en ce que la mesure est effectuée dans un intervalle de temps déterminé à l'avance et en ce qu'on ajuste dans la solution réactionnelle une concentration en agent tensio-actif de 0,01 à 1,5 mmoles/l, une concentration en cholestérolestérase de 0,1 à 30 U/ml et une valeur de pH de 6,5 à 8,0.

2. Procédé suivant la revendication 1, caractérisé en ce qu'on utilise un agent tensio-actif nonionique à une concentration de 0,01 à 0,30 mmole/l.

3. Procédé suivant la revendication 2, caractérisé en ce que la concentration en agent tensio-actif est de 0,06 à 0,18 mmole/l.

4. Procédé suivant la revendication 1, caractérisé en ce qu'on utilise un agent tensio-actif du groupe des acides biliaires à une concentration de 0,01 à 1,5 mmoles/l et en ce que la valeur de pH est maintenue à 7,2 jusqu'à 8,0.

5. Procédé suivant la revendication 4, caractérisé en ce que la concentration en agent tensio-actif est de 0,05 à 1,10 mmoles/l et en ce que la valeur du pH est maintenue à 7,6 jusqu'à 8,0.

6. Procédé suivant la revendication 1, caractérisé en ce qu'on utilise un agent tensio-actif cationique à une concentration de 0,02 à 0,80 mmole/l et en ce que la valeur du pH est ajustée à 6,5 jusqu'à 7,6.

7. Procédé suivant la revendication 6, caractérisé en ce que la concentration en agent tensio-actif est de 0,08 à 0,35 mmole/l et en ce que la valeur du pH est ajustée à 6,8 jusqu'à 7,2.

8. Procédé suivant la revendication 1, caractérisé en ce qu'on utilise un agent tensio-actif anionique à une concentration de 0,01 à 0,70 mmole/l.

9. Procédé suivant la revendication 8, caractérisé en ce que la concentration en agent tensio-actif est de 0,05 à 0,35 mmole/l.

10. Procédé suivant l'une des revendications 1 à 8, caractérisé en ce que la mesure est effectuée dans les 0,5 à 15 minutes après le début de la réaction.

11. Procédé suivant l'une des revendications précédentes, caractérisé en ce qu'on utilise une cholestéroloxydase de Nocardia erythropolis.

12. Procédé suivant l'une des revendications précédentes, caractérisé en ce qu'on utilise une cholestérolestérase de Candida ou de Pseudomonas.

13. Réactif pour l'exécution du procédé suivant la revendication 1, caractérisé en ce qu'il contient
200 à 1000 U/l de cholestéroloxydase,
1000 à 3000 U/l de peroxydase,
2000 à 10 000 U/l de cholestérolestérase,
0,10 à 0,16 mmole/l d'agent tensio-actif,
2 à 20 mmoles/l de phénol,
0,5 à 3 mmoles/l de 4-aminoantipyrine,
70 à 130 mmoles/l de tris/HCl, pH 7,3 à 7,7.

FIG.1a

FIG 1b

SERUM + HDL

FIG 2a

FIG. 2b

FIG. 3a

FIG. 3b

CHOLESTERIN [mg/ 100 ml]

ΔE

LDL–CHOL.

HDL–CHOL.

SERUM + HDL

ΔE

0.200

0.100

0

200

100

0

a    b    c    d